# EUROPEAN PATENT SPECIFICATION

(11) **EP 1 424 976 B1**
(45) Date of publication and mention of the grant of the patent: **17.08.2005**
(21) Application number: 02736410.8
(22) Date of filing: 07.06.2002
(51) Int. Cl.: A61F 13/15

(54) **ABSORBENT DISPOSABLE ARTICLE**
SAUGFÄHIGER EINWEGARTIKEL
ARTICLE ABSORBANT JETABLE

(30) Priority: 25.06.2001 SE 0102246
(43) Date of publication of application: 09.06.2004
(73) Proprietor: SCA Hygiene Products AB, 405 03 Göteborg (SE)
(72) Inventor: ANDERSSON, Mikael, S-416 53 Göteborg (SE); OLSSON, Ken, S-421 35 Västra Frölunda (SE); STORM, Anna-Karin, S-412 75 Göteborg (SE)
(74) Representative: Olsson, Stefan
(86) International application number: PCT/SE2002/001099
(87) International publication number: WO 2003/000164

(56) References cited:
- EP-A2- 0 623 330
- EP-A2- 0 732 094
- EP-A2- 0 951 885
- WO-A1-01/13842
- US-A- 5 575 784

## Description

### TECHNICAL FIELD

The present invention relates to an absorbent disposable article, such as nappy pants, a sanitary towel in the form of briefs or the like, which comprises an inner layer which faces the wearer during use of the article, an outer layer and an absorbent element arranged between the outer and inner layers, in addition to which the article has a crotch portion and, located on both sides of the latter, waist portions which surround the waist of the wearer during use of the article, and in addition to which a tape means is permanently attached by a portion on the outside of said outer layer, said tape means having a tape flap which is arranged in a parked state during use of the article and which can be brought from its parked state into a state of use in order, after use of the article and after folding or rolling together of the latter to form a package, to be usable for closing the package formed.

### BACKGROUND ART

After using sanitary disposable articles, such as nappies and sanitary towels, it is desirable, principally for hygienic reasons but also for aesthetic reasons, to be capable of enclosing the used article in a package in order to prevent faeces, urine or menstrual fluid taken up in the article from leaking out or causing soiling. When a wearer wishes to replace a used article with a new one, it is often not easy to dispose of the used article in a hygienically satisfactory manner if the wearer is not at home. One does not of course always have a refuse bag for used sanitary articles to hand and, if there is a waste bin or the like, it is not satisfactory openly to discard used sanitary articles therein. Hygienic bags for used sanitary towels are included with packs of sanitary towels, but this is not the case as far as the sale of nappies for infants is concerned, and parents and others who are responsible for a toddler must always be mindful of having a refuse bag to hand.
In the use of a certain type of nappy for infants with tape, which for a time was the predominant type of nappy for infants, it was easy to solve the abovementioned problem of handling used nappies by means of the tape which is used for attaching the nappy to the infant. In order for this to function, it is a prerequisite that the tape on a nappy fitted to an infant can be freed without the tape flap taking material along with it on its attachment surface, which material would make it impossible to use the tape again. A nappy with usable attachment tape can be folded or rolled together to form a suitable package which is closed by means of the tape.

In the use of what are known as hook-and-loop connections which are now common, this type of connection often does not function for closing a used nappy to form a package. In order for it to work, it is a prerequisite that the hook-and-loop hooks can be attached to the outer layer of the nappy, which is frequently not the case.

The use of nappy pants has become increasingly common and in these there is of course no tape or the like at all which can be used for closing a used article which has been folded or rolled to form a package for disposal. It has therefore become usual to provide nappies with hook-and-loop connections and also nappy pants with a special tape means which is intended to be used solely for closing folded or rolled-together used nappies or nappy pants. In known solutions, these tape means are positioned on the outside of the article and in the waist portion on the front or rear side of the nappy or nappy pants. The folding together or rolling together of a used article is effected from that end portion of the article which is not provided with said tape means, after which the tape flap is raised from its parked position and is attached to the folded or rolled-together portion.

The use of tape means for closing a folded-together or rolled-together used sanitary article, such as nappy pants, is associated with a great many problems. The adhesion of the tape flap to the outside of the article is of course dependent on the choice of outer material for the article. If the outer layer is made of plastic, a commercially available nappy tape will attach without problems. However, it has been found that consumers prefer sanitary disposable articles, such as nappy pants, which have a textile-like outside, and plastic is then not an alternative, but use is made of various types of non-woven fabrics which can be constituted by one layer of non-woven or consist of a laminate of the same type or different types of non-woven. Said non-woven fabrics are in turn usually laminated with a liquidtight plastic film.

Solutions exist in which the tape means for closing folded-together or rolled-together nappy pants consists of one part in the form of a hook-and-loop connection while the other part consists of either the outside of the article or a special receiving zone which is arranged in a suitable place on the outside of the article in order to interact with the tape means. However, such solutions are relatively expensive and do not solve the particular problems which the present invention solves, namely that the permanent connection of the tape means to the outer layer of the article is sufficiently strong.

A problem which has certainly been taken into account but not solved in a satisfactory manner in the manufacture of previously known sanitary disposable articles with special tape means for closing used articles to form a package for disposal is that, for safety reasons, the tape means has to be strongly permanently anchored on the outside of the article. There is a risk that an infant will work the tape means loose and swallow it.

The object of the present invention is to produce a construction which affords such permanent anchoring of the tape means that there is no risk whatsoever of an infant being able to work the tape means loose from the remainder of the article by pulling the tape flap.

As these products are disposable articles in a fiercely competitive marketplace, the solution must be produced at the lowest possible price. At the same time, other requirements relating to the outer layer of the disposable article have to be satisfied, that is to say it is to be so hardwearing that there is no risk of it breaking during normal use and handling, and at the same time the material is to feel soft and pleasant to the wearer and to afford the appearance and sensation of textile. All these requirements result in it being difficult to find a satisfactory solution.

All known commercially available sanitary disposable articles with special tape means for closing used articles folded or rolled together to form a package have been reviewed in order to determine whether or not the tape means is fixed adequately safely. In this connection, it was established that not one known article we examined was satisfactory from the point of view of safety. An infant would be able to pull the tape means loose and swallow it. The degree of adhesion of the tape means depends on the adhesive capacity of the tape, the choice of outer layer on the disposable article and the construction of the tape. In the articles examined, the resistance to pulling in the longitudinal direction of the tape attachment surface is adequate, that is to say the tape means performs its intended function well. On the other hand, it is easy to free the tape means if the free tape flap end is pulled in the direction towards the opposite, anchored end of the tape means, that is to say the tape is rolled off bit by bit.

From EP 0 826 352 A2, a construction of the tape means is known, which makes this type of pulling-off, that is to say pulling off bit by bit, impossible. The tape means according to said publication has two attachment legs permanently anchored to the outer layer, and the free tape flap end has its attachment where these attachment legs meet and is connected to each of these. If, therefore, pulling takes place in the direction towards the end of one of the permanently anchored attachment legs, no peeling-off of this leg takes place because the force will be taken up in the longitudinal direction of the other leg permanently connected to the outer layer. A tape attachment means can therefore not be freed as easily by an infant as the constructions available on the market. The stresses on an attachment according to said publication are greatest when the tape flap is pulled at right angles out from the plane of the outer layer. EP 0 826 352 A2 provides no indication of how the outer layer is to be designed or how great a stress the tape means will withstand before it comes away. In EP 0 826 352, it is stated that the outer layer consists of a plastic film, which means that the problems in the form of low tape adhesion associated with the use of a non-woven fabric as the outer layer are not addressed.

From WO 01/13 842 A1, an absorbent disposable product with a fastener device provided on the outer layer is known. As backsheet, WO 01/13 842 A1 refers to a laminate of a liquid impervious film joined with an outer cover nonwoven layer. However, the nonwoven layer may be formed by any type of monwoven, material, and in a preferred embodiment a corded nonwoven material

### DISCLOSURE OF INVENTION

According to the invention, an absorbent article of the type mentioned in the introduction is characterized in that the tape means is designed so that it is essentially T-shaped in longitudinal section when the tape flap has been freed from its parked state and raised into an extended state projecting straight out from the outer layer, the tape flap forming a stem portion of said T while the two cross-legs of said T constitute the portion permanently attached to the outer layer, as a result of which tensile forces in the tape flap are taken up either by the two legs or in the longitudinal direction by one leg, in that the outer layer consists of a laminate of non-woven fabric, which laminate contains at least one outer ply of spunbond non-woven and an inner ply of at least one meltblown non-woven, and in that the tape means is permanently attached to the outer layer with such adhesion that the tape means withstands a load of at least 25 N in said tape flap without any risk of the tape means coming away from the outer layer.

Research has shown that an infant is incapable of exerting a tensile force as great as 25 N. This and other desirable criteria, such as textile appearance and textile feel and also low cost, have been achieved by an article having said features.

According to one embodiment, the invention is characterized in that the two legs of the tape means and the movable tape flap have a width of at most 30 mm, suitably at most 20 mm. Restricting the width to this value is suitable from the point of view of cost and also for environmental reasons. Material savings in disposable products are of course especially important for environmental reasons.

According to another illustrative embodiment, the invention is characterized in that the outer layer consists of an SSMMS laminate. It has been found that this laminate affords a very high adhesive power at the same time as it is suitable for the purpose of saving material.

According to another illustrative embodiment, the invention is characterized in that the tape means is permanently attached to the outer layer with such adhesion that the tape flap withstands a load of at least 30 N without any risk of the tape means coming away from the outer layer.

Further embodiments emerge from the other patent claims.

### BRIEF DESCRIPTION OF DRAWINGS

The invention will be described in greater detail below with reference to illustrative embodiments shown in the accompanying drawings, in which
Figure 1 shows diagrammatically the anchoring of a previously known tape means for closing a package consisting of a used absorbent product;
Figure 2 shows diagrammatically the anchoring principle of a tape means forming part of the product according to the present invention;
Figure 3 shows in perspective an absorbent article according to the invention in the form of nappy pants with a tape means intended for closing rolled-together or folded-together nappy pants after use;
Figure 4 shows a longitudinal section of an embodiment of a tape means forming part of a product according to the invention;
Figure 5 shows the nappy pants shown in Figure 3 after use and in the folded-together state, and
Figure 6 shows diagrammatically the method of measuring the force necessary to pull a tape means from its anchoring.

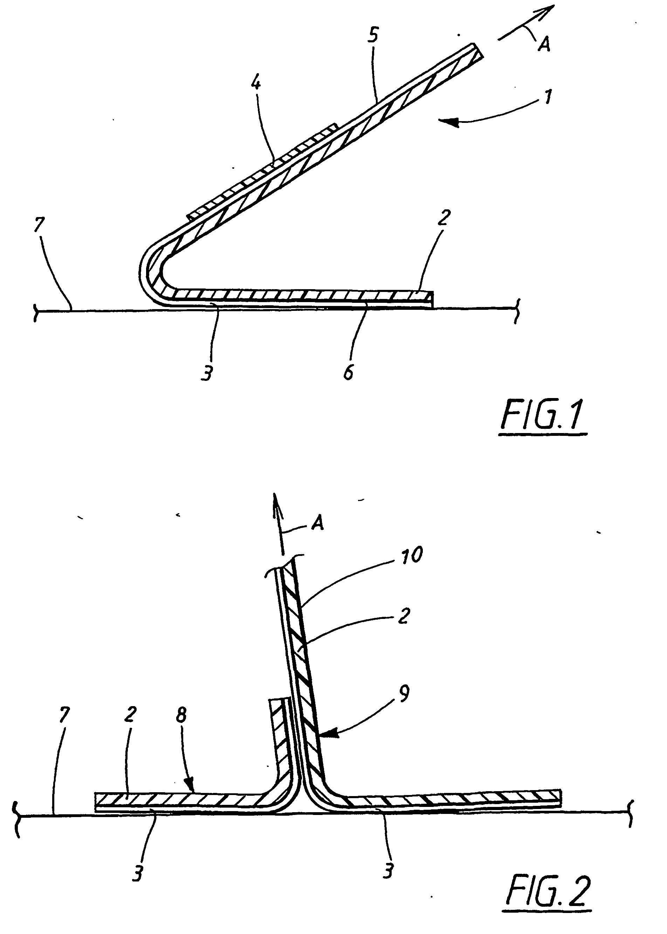

Figure 1 shows a tape means 1 of the type used in commercially available absorbent disposable products, which is intended, after use and folding-together of the product, to close the product in this folded-together state. The tape means 1 consists of a support 2 and an adhesive 3 arranged thereon. The tape means has a layer 4 which is attached to the adhesive 3 and the outside of which is treated with release agent in order that the free end portion 5 provided with adhesive will be capable in a parked state of being attached detachably to said layer 4. The tape means has an end portion 6 provided with adhesive, which is intended to be permanently arranged on the outside 7 of the absorbent product. Figure 1 shows what would occur if, for example, an infant were to take hold of the free end of the tape means 1 and pull in the direction of the arrow A. The portion 6 intended for permanent attachment would then be peeled off bit by bit from the base 7. This is illustrated in Figure 1 by a part portion provided with adhesive having been peeled off from the base to some extent.

Figure 2 illustrates a tape means which, compared with the construction according to Figure 1, is more suitably designed and is of a type which is intended to be used in an absorbent product according to the present invention. The tape means consists of two tape portions, a shorter tape portion 8 and a longer tape portion 9, which consist of a support 2 and an adhesive 3. As can be seen from Figure 2, the two tape portions 8 and 9 are interconnected along part of their extent, adhesive to adhesive. As a result, this connection is very strong. The long tape portion 9 has a free end 10, only a part of which is shown in the drawing. This free end is intended to constitute the tape flap. The tape portions 8 and 9 form two legs, by means of which the tape means is attached to the base 7, that is to say the outside of an absorbent article. The anchoring of the tape means according to Figure 2 is much stronger than the anchoring according to Figure 1. The tape means according to Figure 2 cannot be peeled off easily bit by bit as shown in Figure 1. The most critical case of loading for the anchoring according to Figure 2 is when the free tape flap is pulled at right angles to the base 7, that is to say as illustrated by the arrow A in Figure 2. Even in this case, however, the anchoring is very good compared with a construction according to Figure 1.

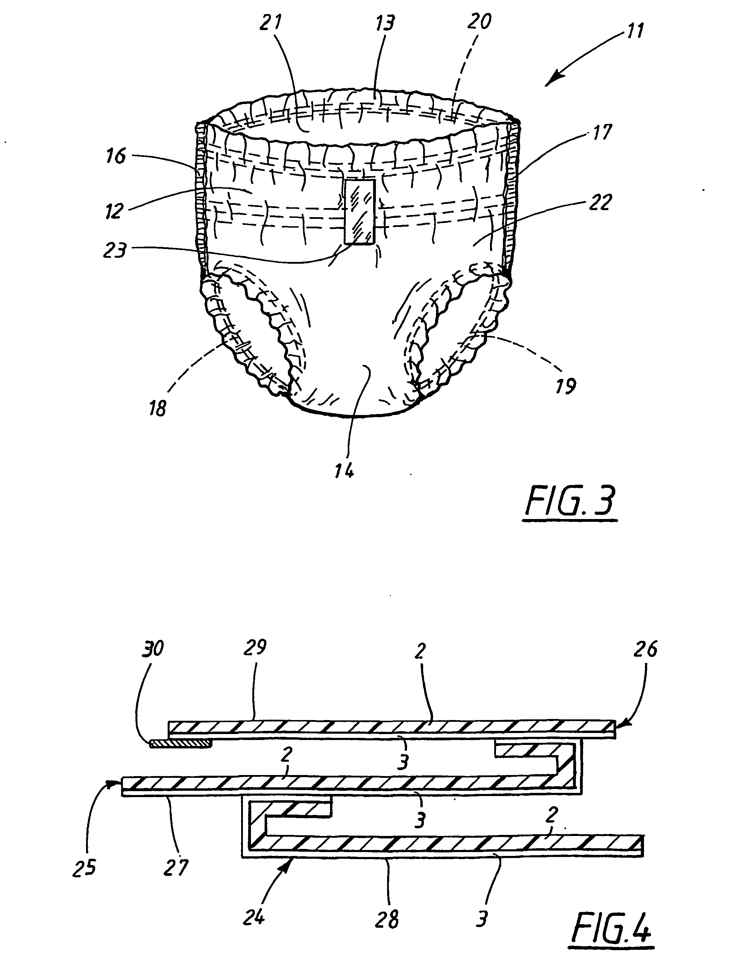

Figure 3 shows nappy pants 11 which consist of two waist portions 12, 13 and also an intermediate crotch portion 14. The waist portions 12, 13 are permanently connected along the edge portions 16, 17. The nappy pants have leg openings with leg elastic 18, 19 arranged around these. The nappy pants are also provided with waist elastic 20 for sealing contact around the waist of the wearer. The nappy pants have an inner layer 21 in contact with the wearer and an outer layer 22 and also have an absorbent element (not shown) arranged between said layers.

A tape means 23 is arranged in the middle of the front waist portion 12. The tape means is of a construction which is described in greater detail below with reference to Figure 4 and is shown in Figure 3 in a parked state, from which a tape flap can be pulled out in the upward direction over and past the front waist portion 12 in order to be capable, after the used nappy pants have been folded together or rolled together, of closing the package formed by connecting the front waist portion to a portion of the outer layer on the opposite side of the waist opening.

In the embodiment shown, the tape means has been arranged on the front waist portion 12 for the sake of clarity. However, it is more suitable to position the tape means permanently on the rear waist portion because it is more difficult for an infant to take hold of the tape and play with it if the tape means is permanently arranged at the rear of the nappy pants.

Figure 4 shows in detail an example of a tape means of the type shown in Figure 3. Here, the tape means is constructed from three tape portions 24, 25 and 26. As in the tape means described above, the different tape portions consist of a support 2 and an adhesive 3. The portions 27 and 28 form the two legs which are intended to be connected permanently to the outer layer 22 of the nappy pants in Figure 3, that is to say to be fixed permanently to the base 7 in the same manner as the legs in the basic design shown in Figure 2. The remaining part of the tape portion 25 and the tape portion 26 together form the free tape flap 29 which is shown in its parked state in Figure 4. In this state, the tape means is as a whole Z-folded, and the outer tape portion 26 is attached by its side provided with adhesive to the support 2 on the tape portion 25, while that part of the tape flap 29 formed by the tape portion 25 is attached detachably to the support 2 on the tape portion 24. In order to facilitate freeing of the tape flap from its parked state, a strip 30 has been arranged on the free end portion of the tape flap.

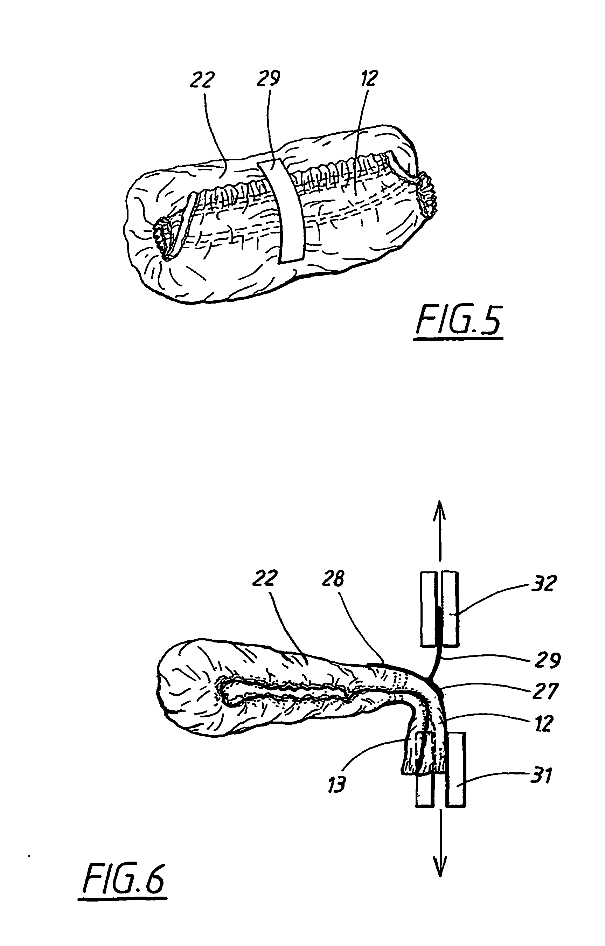

Figure 5 shows how folded-together used nappy pants have been secured in the folded-together state by means of the tape flap 29 which has been raised from its parked state and extended from the front waist portion of the nappy pants over the waist opening and attached to the outer layer 22 in a place on the opposite side of the waist opening of the nappy pants.

Research has shown that infants are incapable of exerting tensile forces exceeding the order of 25 N. A tape means according to the present invention is therefore permanently anchored to the outer layer of the nappy pants with such adhesion that the tape means can take up a load in the tape flap of at least 25 N irrespective of the direction in which the tensile force is exerted.

Figure 6 illustrates the method of measuring the load on the tape means. The measurements are performed in a tensile tester, for example of the Instron brand (supplied by Instron Corporation). Figure 6 shows the fastening of nappy pants according to Figure 3 in the tensile tester for measuring the load to which the tape flap 29 can be subjected without the tape means coming away from its permanent attachment to the outer layer of the nappy pants. Only the waist portion 12, on which the tape means is permanently arranged, is fastened in the lower clamp 31 of the tensile tester. Only the covering with elastic and not the absorbent element is to be fastened in said lower clamp. The tape flap 29 is pulled out and fastened in the upper clamp 32 of the tensile tester. When pulling starts, the nappy pants are, as can be seen in Figure 6, to be at right angles to the pulling direction. The maximum value when the tape means comes away from the outer layer is recorded. The width of the lower clamp should be at least 200 mm, and the speed of the tensile tester should be 300 mm/min.

For good adhesion to the tape, the outer layer is made from a laminate consisting of, from the outside, at least one layer of spunbond (S), at least one layer of meltblown (M) and, on the inside, a layer of spunbond (S). It is previously known to use what are known as non-wovens in the form of laminates of a number of layers of spunbond and meltblown, for example SMS laminate, SMMS laminate etc., as the outer layer on absorbent articles, such as nappies and the like. According to the present invention, the importance is emphasized of using precisely this type of outer layer in order to obtain particularly good adhesion of tape means of the type used on absorbent disposable articles at the same time as the laminate in question is soft and has a textile feel and textile appearance. Such a material is moreover cost-effective compared with other materials with corresponding strength properties.

An example of a suitable outer layer on an absorbent article according to the present invention is an SSMMS laminate from Fibertex with the designation H201010702 with weights per unit area S/S/M/M/S equal to 4.7/4.7/1.5/1.5/4.6 g/m², that is to say with a total weight per unit area of 17 g/m².

For reasons of cost and environmental reasons, the tape means should have a width which does not exceed 30 mm and preferably does not exceed 20 mm.

The invention is not limited to the illustrative embodiments described above, but a large number of modifications are possible within the scope of the following patent claims.

The tape means does not have to be designed as described above but can be folded in a manner other than in a Z-shape before use. The tape flap of the tape means can be provided with a stretchable portion to facilitate its extension.

The tape means can be designed in many ways. The essential feature is that the tape means is designed so that, when the tape flap has been freed and is extended at right angles to the outer layer, it forms the shape of a T in longitudinal section with the two cross-legs permanently connected to the outer layer and the tape flap constituting the stem portion of said T-shape, as a result of which loads arising in the tape flap are taken up by the two cross-legs.

## Claims

1. Absorbent disposable article, such as nappy pants, a sanitary towel in the form of briefs or the like, which comprises an inner layer (21) which faces the wearer during use of the article, an outer layer (22) and an absorbent element arranged between the outer and inner layers, in addition to which the article has a crotch portion (14) and, located on both sides of the latter, waist portions (12, 13) which surround the waist of the wearer during use of the article, and in addition to which a tape means (23) is permanently attached by a portion on the outside of said outer layer (22), said tape means having a tape flap (29) which is arranged in a parked state during use of the article and which can be brought from its parked state into a state of use in order, after use of the article and after folding or rolling together of the latter to form a package, to be usable for closing the package formed, **characterized in that** the tape means (23) is designed so that it is essentially T-shaped in longitudinal section when the tape flap (29) has been freed from its parked state and raised into an extended state projecting straight out from the outer layer (22), the tape flap forming a stem portion of said T while the two cross-legs (27, 28) of said T constitute the portion permanently attached to the outer layer (22), as a result of which tensile forces in the tape flap (29) are taken up either by the two legs (27, 28) or in the longitudinal direction by one leg (27 or 28), **in that** the outer layer (22) consists of a laminate of nonwoven fabric, which laminate contains at least one outer ply of spunbond non-woven and an inner ply of at least one meltblown non-woven, and **in that** the tape means (23) is permanently attached to the outer layer (22) with such adhesion that the tape means withstands a load of at least 25 N in said tape flap (29) without any risk of the tape means coming away from the outer layer.

2. Article according to Claim 1, **characterized in that** the two legs (27, 28) of the tape means (23) and the movable tape flap (29) have a width of at most 30 mm, suitably at most 20 mm.

3. Article according to Claim 1 or 2, **characterized in that** the outer layer (22) consists of an SMS laminate.

4. Article according to Claim 1 or 2, **characterized in that** the outer layer (22) consists of an SMMS laminate.

5. Article according to Claim 1 or 2, **characterized in that** the outer layer (22) consists of an SSMMS laminate.

6. Article according to any one of the preceding claims, **characterized in that** the tape flap (29) is in its parked state folded along a fold line across the tape flap in at least one place along the length of the tape flap in addition to the fold line at the transition to the permanently attached legs (27, 28) of the tape means and attached detachably to the legs of the tape means by the attachment adhesive (3) of the tape flap.

7. Article according to Claim 6, **characterized in that**, in addition to said attachment adhesive (3), which is intended for closing a folded or rolled-together used article, the tape flap (29) has at least one further adhesive area which is intended to hold the tape flap detachably in its folded parked state in contact with the legs of the tape means.

8. Article according to any one of the preceding claims, **characterized in that** the tape flap has a stretchable portion which makes it possible to lengthen the tape flap when closing a folded-together or rolled-together used article.

9. Article according to any one of the preceding claims, **characterized in that** the tape means (23) is permanently attached to the outer layer (22) with such adhesion that the tape flap (29) withstands a load of at least 30 N without any risk of the tape means coming away from the outer layer.

## Patentansprüche

1. Absorbierender Wegwerfgegenstand, wie beispielsweise Höschenwindeln, eine Binde in Form einer Unterhose oder ähnliches, der eine innere Schicht (21), die dem Träger während der Verwendung des Gegenstands zugewandt ist, eine äußere Schicht (22) und ein zwischen der äußeren und inneren Schicht angeordnetes absorbierendes Element umfasst, wobei der Gegenstand zusätzlich einen Schrittabschnitt (14) und an beiden Seiten des letztgenannten angeordnete Taillenabschnitte (12, 13), die während der Verwendung des Gegenstands die Taille des Trägers umgeben, aufweist, und wobei zusätzlich durch einen Abschnitt auf der Außenseite der äußeren Schicht (22) eine Befestigungseinrichtung (23) permanent angebracht ist, wobei die Befestigungseinrichtung eine Befestigungslasche (29) aufweist, die während der Verwendung des Gegenstands in einem geparkten Zustand angeordnet und die aus diesem geparkten Zustand in einen Verwendungszustand gebracht werden kann, nachdem der Gegenstand verwendet wurde und nachdem letzterer zusammengefaltet oder zusammengerollt wurde, um eine Packung zu bilden, um zum Verschließen der gebildeten Packung verwendbar zu sein, **dadurch gekennzeichnet, dass** die Befestigungseinrichtung (23) derart ausgestaltet ist, dass sie im Längsschnitt im Wesentlichen T-förmig ist, wenn die Befestigungslasche (29) aus dem geparkten Zustand befreit wurde und in einen ausgestreckten Zustand aufgerichtet wurde, indem sie von der äußeren Schicht (22) gerade herausragt, wobei die Befestigungslasche den Stielabschnitt des T bildet, während die zwei Querschenkel (27, 28) des T den Abschnitt bilden, der permanent an der äußeren Schicht (22) angebracht ist, wodurch Zugkräfte in der Befestigungslasche entweder durch die zwei Schenkel (27, 28) oder in der Längsrichtung durch einen Schenkel (27 oder 28) aufgenommen werden, dadurch, dass die äußere Schicht (22) aus einem Laminat aus Vliesstoff besteht, welches Laminat wenigstens eine äußere Lage aus Spunbond-Vliesstoff und eine innere Lage aus wenigstens einem Meltblown-Vliesstoff enthält, und dadurch, dass die Befestigungseinrichtung (23) an der äußeren Schicht (22) mit einer solchen Haftwirkung permanent angebracht ist, dass die Befestigungseinrichtung einer Last von wenigstens 25 N an der Befestigungslasche (29) standhält, ohne jegliches Risiko, dass sich die Befestigungseinrichtung von der äußeren Schicht löst.

2. Gegenstand nach Anspruch 1, **dadurch gekennzeichnet, dass** die zwei Schenkel (27, 28) der Befestigungseinrichtung (23) und der bewegbaren Befestigungslasche (29) eine Breite von höchstens 30 mm, geeigneterweise höchstens 20 mm, aufweisen.

3. Gegenstand nach Anspruch 1 oder 2, **dadurch gekennzeichnet, dass** die äußere Schicht (22) aus einem SMS-Laminat besteht.

4. Gegenstand nach Anspruch 1 oder 2, **dadurch gekennzeichnet, dass** die äußere Schicht (22) aus einem SMMS-Laminat besteht.

5. Gegenstand nach Anspruch 1 oder 2, **dadurch gekennzeichnet, dass** die äußere Schicht (22) aus einem SSMMS-Laminat besteht.

6. Gegenstand nach einem der vorstehenden Ansprüche, **dadurch gekennzeichnet, dass** die Befestigungslasche (29) in ihrem geparkten Zustand entlang einer Faltlinie quer über die Befestigungslasche an wenigstens einer Stelle entlang der Länge der Befestigungslasche zusätzlich zu der Faltlinie am Übergang zu den permanent angebrachten Schenkeln (27, 28) der Befestigungseinrichtung gefaltet ist und an den Schenkeln der Befestigungseinrichtung mittels des Befestigungsklebemittels (3) der Befestigungslasche lösbar angebracht ist.

7. Gegenstand nach Anspruch 6, **dadurch gekennzeichnet, dass** zusätzlich zu dem Befestigungsklebemittel (3), das zum Verschließen eines zusammengefalteten oder zusammengerollten verwendeten Gegenstands gedacht ist, die Befestigungslasche (29) wenigstens einen weiteren Klebemittelbereich aufweist, der dazu gedacht ist, die Befestigungslasche in ihrem gefalteten geparkten Zustand in Kontakt mit den Schenkeln der Befestigungseinrichtung zu halten.

8. Gegenstand nach einem der vorstehenden Ansprüche, **dadurch gekennzeichnet, dass** die Befestigungslasche einen dehnbaren Abschnitt aufweist, der es ermöglicht, die Befestigungslasche zu verlängern, wenn ein zusammengefalteter oder zusammengerollter verwendeter Gegenstand verschlossen wird.

9. Gegenstand nach einem der vorstehenden Ansprüche, **dadurch gekennzeichnet, dass** die Befestigungseinrichtung (23) permanent an der äußeren Schicht (22) mit einer solchen Haftwirkung angebracht ist, dass die Befestigungslasche (29) einer Last von wenigstens 30 N standhält, ohne jegliches Risiko, dass sich die Befestigungseinrichtung von der äußeren Schicht löst.

## Revendications

1. Article absorbant jetable, tel qu'une couche-culotte, une serviette hygiénique sous la forme d'une culotte ou similaire, qui comprend une couche intérieure (21) tournée vers l'utilisateur pendant l'utilisation de l'article, une couche extérieure (22) et un élément absorbant placé entre les couches intérieure et extérieure, l'article comportant en outre une partie entrejambe (14) et, de chaque côté de cette dernière, des parties ceinture (12, 13) qui entourent la taille de l'utilisateur pendant l'utilisation de l'article, un moyen formant bande (23) étant en outre attaché de manière permanente par une partie sur l'extérieur de ladite couche extérieure (22), ledit moyen formant bande comportant un rabat de bande (29) qui est agencé en un état de rangement pendant l'utilisation de l'article et que l'on peut faire passer de l'état de rangement à un état d'utilisation, après l'utilisation de l'article et après avoir plié ou roulé ce dernier pour former un paquet, afin qu'il puisse être utilisé pour fermer le paquet formé, **caractérisé en ce que** le moyen formant bande (23) est dessiné de telle manière qu'il est essentiellement en forme de T en section longitudinale lorsque le rabat de bande (29) a été libéré de son état de rangement et levé dans un état étendu faisant saillie tout droit hors de la couche extérieure (22), le rabat de bande formant une partie pied dudit T tandis que les deux branches croisées (27, 28) dudit T constituent la partie attachée de façon permanente à la couche extérieure (22), en conséquence de quoi les forces de traction présentes dans le rabat de bande (29) sont encaissées soit par les deux branches (27, 28), soit dans la direction longitudinale par une branche (27 ou 28), **en ce que** la couche extérieure (22) est constituée d'un stratifié de voile non tissé, lequel stratifié contient au moins une nappe extérieure de non tissé filé-lié et une nappe intérieure d'au moins un non tissé fondu-soufflé, et **en ce que** le moyen formant bande (23) est fixé de manière permanente à la couche extérieure (22) avec une adhérence telle que le moyen formant bande supporte une charge d'au moins 25 N dans ledit rabat de bande (29) sans aucun risque que le moyen formant bande ne se détache de la couche extérieure.

2. Article selon la revendication 1, **caractérisé en ce que** les deux branches (27, 28) du moyen formant bande (23) et le rabat de bande mobile (29) ont une largeur d'au plus 30 mm, de préférence d'au plus 20 mm.

3. Article selon la revendication 1 ou 2, **caractérisé en ce que** la couche extérieure (22) est constituée d'un stratifié SMS.

4. Article selon la revendication 1 ou 2, **caractérisé en ce que** la couche extérieure (22) est constituée d'un stratifié SMMS.

5. Article selon la revendication 1 ou 2, **caractérisé en ce que** la couche extérieure (22) est constituée d'un stratifié SSMMS.

6. Article selon l'une quelconque des revendications précédentes, **caractérisé en ce que** le rabat de bande (29) est, dans son état de rangement, plié le long d'une ligne de pliage qui va d'un bord à l'autre du rabat de bande en au moins un endroit sur la longueur du rabat de bande en plus de la ligne de pliage au niveau de la transition avec les branches fixées de façon permanente (27, 28) du moyen formant bande et fixé de manière détachable aux branches du moyen formant bande par l'adhésif de fixation (3) du rabat de bande.

7. Article selon la revendication 6, **caractérisé en ce que**, en plus dudit adhésif de fixation (3), qui est prévu pour fermer un article usagé plié ou roulé, le rabat de bande (29) comporte au moins une zone adhésive supplémentaire qui est destinée à maintenir de manière détachable le rabat de bande dans son étant de rangement plié en contact avec les branches du moyen formant bande.

8. Article selon l'une quelconque des revendications précédentes, **caractérisé en ce que** le rabat de bande comporte une partie extensible qui permet de rallonger le rabat de bande lors de la fermeture d'un article usagé plié ou roulé.

9. Article selon l'une quelconque des revendications précédentes, **caractérisé en ce que** le moyen formant bande (23) est fixé de manière permanente à la couche extérieure (22) avec une adhérence telle que le rabat de bande (29) supporte une charge d'au moins 30 N sans aucun risque que le moyen formant bande ne se détache de la couche extérieure.
